# EUROPEAN PATENT SPECIFICATION

(11) **EP 1 198 465 B1**
(45) Date of publication and mention of the grant of the patent: **08.09.2004**
(21) Application number: 00931478.2
(22) Date of filing: 07.06.2000
(51) Int. Cl.: C07D 491/052

(54) **PROCESS FOR THE PREPARATION OF 1,8-DISUBSTITUTED-1,3,4,9-TETRAHYDROPYRANO (3,4-B)-INDOLE-1-ACETIC ACID ESTERS IN A HYDROXYLIC SOLVENT**
VERFAHREN ZUR HERSTELLUNG VON 1,8-DISUBSTITUIERTEN-1,3,4,9-TETRAHYDROPYRAN(3,4-B)-INDOL-1ESSIGSÄUREESTERN IN EINEM HYDROXYLHALTIGEN LÖSUNGSMITTEL
PROCEDE DE PREPARATION DES ESTERS ACETIQUES 1,8-DISUBSTITUES DE 1,3,4,9-TETRAHYDROPYRANO (3,4-B)-INDOLE-1 DANS UN SOLVANT HYDROXYLIQUE

(30) Priority: 11.06.1999 IN DE086599; 04.10.1999 US 412455
(43) Date of publication of application: 24.04.2002
(73) Proprietor: RANBAXY LABORATORIES, LTD., New Delhi 110 019 (IN)
(72) Inventor: VIJAYARAGHAVAN, B., Gurgaon-122001, Haryana (IN); RAMANA, K., V., Gurgaon 122 001, Haryana (IN); KHERA, Brij, New Delhi 110 015 (IN); KUMAR, Naresh, Gurgaon 122 001, Haryana (IN)
(74) Representative: Cronin, Brian Harold John
(86) International application number: PCT/IB2000/000760
(87) International publication number: WO 2000/077006

(56) References cited:
- US-A- 3 939 178
- US-A- 4 012 417
- US-A- 4 309 348
- US-A- 4 585 877
- COSTA P R R ET AL: "Asymmetric Friedel-Crafts Reaction Mediated by New Chiral Auxiliaries Derived From (1S)-( ?)-beta-Pinene: Enantioselective Synthesis of ( ?)-8-Norethyl, 1 ?-Normethyl Etodolac" TETRAHEDRON LETTERS,NL,ELSEVIER SCIENCE PUBLISHERS, AMSTERDAM, vol. 38, no. 40, 6 October 1997 (1997-10-06), pages 7021-7024, XP004090409 ISSN: 0040-4039

## Description

### FIELD OF THE INVENTION

This invention relates to a process for the preparation of the esters of 1,8-disubstituted -1,3,4,9-tetrahydropyrano (3,4-b)-indole-1-acetic acid.

### BACKGROUND OF THE INVENTION

The esters of the present invention are used as intermediates for the preparation of the corresponding 1,8-dialkyl-1-3,4,9-tetrahydropyrano (3,4-b)-indole-1-acetic acids, which include etodolac, an anti-inflammatory and analgesic compound reported first by Demerson et al., U.S. Patent No. 3,939,178.

Throughout, preparation of these esters has been previously described in U.S. Patent Nos. 3,939,178; 4,012,417; 4,585,877; and several related patents. These esters are prepared from the corresponding 7-substituted tryptophol and alkyl propionylacetate. U.S. Patent Nos. 3,939,178 and 4,012,417 disclose reaction of substituted tryptophols with keto ester to produce pyrano (3,4-b) indoles. Suitable solvents described are benzene, toluene, diethyl ether, dioxan, tetrahydrofuran, methylene dichloride, carbon tetrachloride and the like. Benzene and tetrahydrofuran are the preferred solvents.

Suitable acid catalysts which may be used for this condensation are the type of catalysts used in Fischer Indole Synthesis and include p-toluenesulfonic acid, phosphorus pentoxide, boron trifluoride, zinc chloride, hydrochloric acid and sulfuric acid and the like. Preferred catalysts being p-toluenesulfonic acid, boron trifluride and phosphorus pentoxide. According to Example 477 of U.S. Patent No. 3,939,178, etodolac ethyl ester is produced by the reaction of 7-ethyltryptophol and the keto ester, ethyl propionyl acetate, using benzene as a solvent and p-toluenesulfonic acid as the catalysts. The product so obtained is purified by column chromatography, which is hydrolysed under alkaline conditions to give etodolac.

U.S. Patent No. 4,585,877 discloses reaction of the methyl ester of 3-methoxy-2-pentenoic acid with 7-ethyltriptophol using dichlaromethane as a solvent and boron trifluoride ethereate as the catalyst.

Tetrahedron Lett. 38, 7021 (1997) discloses a process for the production of 1,8-disubstituted esters of 1,3,4,9-tetrahydro-pyrano (3,4-b)-indole-1-acetic acid of formula I, wherein the reaction is made possible using THF and BF₃.Et₂O as solvent/catalyst.

U.S. Patent No. 4,309,348 discloses indole derivatives characterized by having a 1,3,4,9-tetrahydropyrano[3,4-b]indole or 1,3,4,9-tetrahydrothiopyrano[3,4-b]indole nucleus with a hydroxyalkanamine or lower alkoxyalkanamine substitutent and a lower alkyl group at position 1. Methods for the preparation of such derivatives are also disclosed.

The methods described in the prior art suffer from several disadvantages:

Firstly, the use of strong acid catalysts exposes the desired product to other acid catalysed reactions resulting in the formation of impurities. In order to minimise the impurities, the ester is either purified by column chromatography before hydrolysis or the hydrolyzed product is purified at the cost of the yields and overall efficiency of the process. Moreover, the purification by column chromatography is very cumbersome and is disadvantageous at a commercial scale because of its efficiency and higher manufacturing costs.

Secondly, the use of carcinogenic solvents like benzene, tetrahydrofuran and strong acids like p-toluenefonic acid, boron trifluoride ethereate or sulfuric acid at industrial scale involves health hazards and poses serious environmental problems.

For purposes of patient safety, it is highly desirable to limit the amount of residual solvent present in any medicament administered to a patient. With this objective in mind, International Regulatory bodies impose stringent limits on residual solvents and categorize solvents in various classes depending upon their toxicity. The solvents used in prior art have been categorized in Class I category, which are highly toxic.

Therefore, the aim of the present invention is to provide an efficient process for the preparation of the esters of 1,8-disubstituted - 1,3,4,9-tetrahydropyrano (3, 4,6)-indole-1-acetic acid of formula I (as shown in the accompanied drawings), which process avoids the use of corrosive solvents, strong corrosive acids and gives a product of high purity and yield.

In general, molar equivalent proportions of 7-alkyltryptophol and B-ketoester is used. More preferably, 0.9-1.2 molar ratio of B-keto ester with respect to 7-alkyltriptophol is used but varying amounts of molar ratios are within the scope of this invention.

### SUMMARY OF THE INVENTION

The present invention provides a process for the preparation of the esters of 1, 8-disubstituted -1,3,4,9-tetrahydropyrane (3,4-b)-indole-1-acetic acid of Formula I: 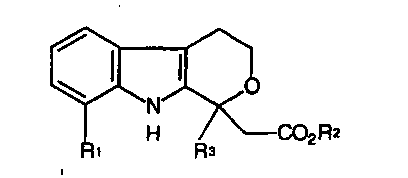 wherein R₁, R₂ and R₃ have the meanings as defined above, said process comprising reacting 7-alkyltryptophol of Formula II: 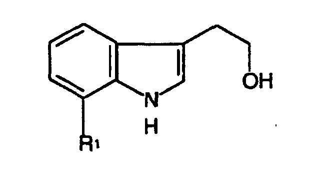 with a β-ketoester of the Formula III: 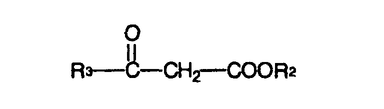 in a hydroxylic solvent, comprising at least one alkanol solvent having 1-4 carbon atoms, containing hydrogen chloride gas, and subsequently recovering the product of Formula I.

The term hydroxylic solvent means any lower alkanol and includes those primary secondary and tertiary alcohols. Suitable lower alkanol solvents include metanol, ethanol, n-propanol, isopropanol, n-butanol, isobutanol and t-butanol. Preferably, methanol is used as a solvent. Mixture of two or more lower alkanols can also be used.

The concentration of hydrogen chloride gas may range from 1% w/v to 20% w/v. However, a range of 5% to 15% w/v is generally preferred. The amount of solvent is at least 1 part by volume per part of the starting material, 7-substituted tryptophol. Higher amounts of solvents and generally upto 20 parts by volume can be used. However, a range of 5-10 parts by volume is generally preferred. Amounts higher than 20 parts are not useful from an economic point of view because large size reactor would be required.

Generally, the reaction is carried out in a temperature range from about -20ºC to the boiling point of the reaction mixture. The preferred temperature range is between about 0ºC to about 30ºC. Persons skilled in the art will appreciate that the temperature range is given as a guide and may vary with the choice of solvent.

The reaction is typically accomplished within about 3-8 hours. However, the length of time required for reaction may vary depending on such factors as temperature of the reaction, volume of reaction mixture and size of batch and container and presence or absence of stirring.

In general, the reaction product directly crystallizes out of the reaction medium and is recovered by filtration to get substantially pure product of Formula I. Methods known in the art may be used with the process of this invention to enhance any aspect of this process. For example, the reaction mixture may be extracted with a solvent such as pentane or hexane. Such a solvent has the characteristic that the desired product is soluble in it and it does not form a homogeneous mixture with a hydroxylic solvent and the solvent is then removed to get the desired product. The product so obtained is hydrolysed by using methods known in the art to get the corresponding acid, e.g. etodolac.

### DETAILED DESCRIPTION OF THE INVENTION

The present invention is illustrated by, but is by no means limited to, the following examples:

### EXAMPLE 1

### Preparation of Methyl 1,8-diethyl-1,3,4,9-tetrahydropropyrano(3,4-b)-indole-1-acetate

To a solution of 7-ethyltryptophol (12.0g) in methanolic hydrogen chloride (5% w/v, 100.0 ml), methyl 3-oxopentanoate (9.1 g) was added in one lot, at 25ºC. The reaction mixture was stirred at 25-30ºC for 7 hours. The reaction mixture was then cooled to 0ºC, and stirred at 0-5ºC for 3 hours. The product was filtered, washed with methanol and dried to afford the title compound (16.7g, 89%), m.p. 128-130ºC; purity : 99.7%.

### EXAMPLE 2

### Preparation of Methyl 8-methyl-1-n-propyl-1,3,4,9-tetrahydropyrano (3,4-b)-indote-1-acetate

To a solution of 7-methyltryptophol (10.0g) in methanolic hydrogen chloride (5% w/v, 100.0 ml), methyl 3-oxohexanoate (9.1g) was added in one lot, at 25ºC. The reaction mixture was stirred at 25-30ºC for 7 hours. The reaction mixture was extracted with hexane (100.0 ml). Hexane was concentrated to about 20ml and methanol (10 ml) was added. The mixture was stirred at 5-10ºC for 2 hours. The product was filtered, washed with methanol, and dried to afford the title compound (13.7g, 80%); purity: 98.1%.

### EXAMPLE 3

### Preparation of Methyl 1, 8-diethyl-1,3,4,9-tetrahydropyrano (3,4-b)-indole-1-acetate

To a solution of 7-ethyltryptophol (12.0g) in 1-butanolic hydrogen chloride (5% w/v, 100.0 ml), methyl 3-oxopentanoate (9.1 g) was added in one lot, at 25ºC. The reaction mixture was stirred at 25-30ºC for 7 hours. The reaction mixture was extracted with hexane (100.0 ml). Hexane was concentrated to about 20 ml and methanol (10 ml) was added. The product was filtered, washed with methanol and dried to afford the titled compound (14.7 g, 79%), m.p. 128-130ºC; purity : 96.2%.

### EXAMPLE 4

### Preparation of Methyl 1,8-diethyl-1,3,4,9-tetrahydropyrano(3,4-b)-indole-1-acetate

To a solution of 7-ethyltryptophol (12.0g) in a mixture of methanolic hydrogen chloride (10% w/v, 50.0 ml) and toluene (50ml), methyl 3-oxopentanoate (9.1g, 0.07 mole) was added in one lot, at 25ºC. The reaction mixture was stirred at 25-30ºc for 7 hours. The reaction mixture concentrated to about 20 ml under reduced pressure. Methanol (50 ml) was added to the concentrate and the solution was extracted with hexane (100ml). Hexane was concentrated to about 20 ml and methanol (10 ml) was added. The product was filtered, washed with methanol and dried to afford the title compound (15.8 g, 84%), m.p. 128-130ºC, purity: 97.8%.

While the present invention has been described in terms of its specific embodiments, certain modifications and equivalents will be apparent to those skilled in the art and are intended to be included within the scope of the present invention.

## Claims

1. A process from the preparation of the esters of 1,8-disubstituted 1,3,4,9-tetrahydropyrano (3,4-b) - indole-1-acetic acid of Formula I: 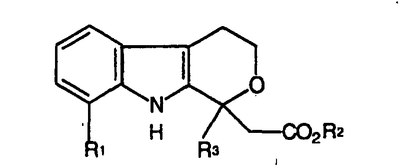 wherein R₁ is selected from the group consisting of hydrogen, lower alkyl and lower alkenyl, R₂ is selected from the group consisting of lower alkyl and aralkyl, R₃ is selected from the group consisting of lower alkyl, lower alkenyl, lower cyclohexyl, phenyl and benzyl, said process comprising reacting 7-alkyltryptophol of Formula II: 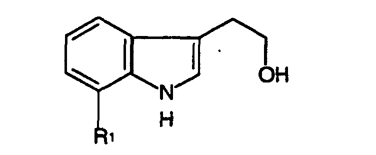 with a β-Ketoester of Formula III: 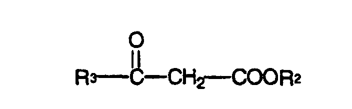 in a hydroxylic solvent, comprising at least one alkanol system having 1-4 carbon atoms, containing hydrogen chloride gas, and subsequently recovering the product of Formula I.

2. The process of claim 1, wherein R₂ is lower alkyl in the compound of Formula I.

3. The process of claim 2, wherein R₂ is methyl in the compound of Formula I.

4. The process of claim 3, wherein R₁ is lower alkyl in the compound of Formula I.

5. The process of claim 4, wherein R₁ is ethyl in the compound of Formula 4.

6. The process of claim 5, wherein R₃ is lower alkyl in the compound of Formula I.

7. The process of claim 6, wherein R₃ is ethyl in the compound of Formula I.

8. The process of claim 1 wherein the hydroxylic solvent comprises a lower alkanol.

9. The process of claim 1 wherein the hydroxylic solvent is methanol, ethanol, n-propanol, isopropanol, n-butanol, or t-butanol.

10. The process according to claim 1 wherein the concentration of hydrogen chloride gas in a hydroxylic solvent is 1-20% w/v.

11. The process according to claim 10 wherein the concentration of hydrogen chloride gas in a hydroxylic solvent is 5-15% w/v.

12. The process according to claim 1 wherein the reaction temperature is in the range -20ºC to 80ºC.

13. The process according to claim 12 wherein the reaction temperature is in the range 0ºC to 30ºC.

14. The process of claim 1 wherein said hydroxylic solvent contains hydrogen chloride gas at the time said reactants are dissolved therein.

15. The process of claim 1 wherein said hydrogen chloride gas is added to the reaction mixture after said reactants are dissolved in said hydroxylic solvent.

16. The process of claim 1 wherein the esters of 1,8-disubstituted 1,3,4,9-tetrahydropyrano(3,4-b)-indole-1-acetic acid of Formula I are recovered by filtration.

17. The process of claim 1 wherein the esters of 1,8-disubstituted 1,3,4,9-tetrahydropyrano(3,4-b)-indole-1-acetic acid of Formula I are recovered by extracting the reaction mixture with a solvent, wherein said solvent has the characteristic that the desired product of Formula I is soluble in it and it does not form a homogeneous mixture with said hydroxylic solvent, and subsequently recovering the product of Formula I by removing the solvent.

18. The process of claim 17 wherein said solvent is selected from pentane and hexane.

19. Use of the process of claim 1 for the preparation of the esters of 1,8-disubstituted 1,3,4,9-tetrahydropyrano(3,4-b)-indole-1-acetic acid of Formula I and their subsequent hydrolysis to give the corresponding acids such as etodolac.

## Patentansprüche

1. Verfahren zur Herstellung von 1,8-disubstituierten 1,3,4,9-Tetrahydropyran(3,4-b)-indol-1-Essigsäureestern der Formel I: 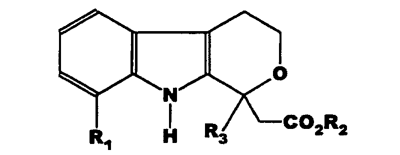 wobei R₁ ausgewählt ist aus der Gruppe bestehend aus Wasserstoff, Niederalkyl und Niederalkenyl, R₂ ausgewählt ist aus der Gruppe bestehend aus Niederalkyl und Aralkyl, R₃ ausgewählt ist aus der Gruppe bestehend aus Niederalkyl, Niederalkenyl, Niedercyclohexyl, Phenyl und Benzyl, wobei besagtes Verfahren die Umsetzung von 7-Alkyltryptophol der Formel II: 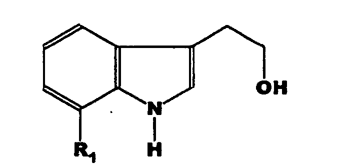 mit einem β-Ketoester der Formel III: 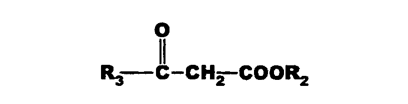 in einem hydroxylhaltigen Lösungsmittel, das mindestens ein Alkanolsystem mit 1-4 Kohlenstoffatomen umfasst und Chlorwasserstoff in Gasform enthält, und die anschließende Wiedergewinnung des Produkts gemäß Formel I umfasst.

2. Verfahren gemäß Anspruch 1, wobei R₂ eine Niederalkylgruppe in der Verbindung gemäß Formel I ist.

3. Verfahren gemäß Anspruch 3, wobei R₂ eine Methylgruppe in der Verbindung gemäß Formel I ist.

4. Verfahren gemäß Anspruch 2, wobei R₁ eine Niederalkylgruppe in der Verbindung gemäß Formel I ist.

5. Verfahren gemäß Anspruch 4, wobei R₁ eine Ethylgruppe in der Verbindung gemäß Formel I ist.

6. Verfahren gemäß Anspruch 5, wobei R₃ eine Niederalkylgruppe in der Verbindung gemäß Formel I ist.

7. Verfahren gemäß Anspruch 6, wobei R₃ eine Ethylgruppe in der Verbindung gemäß Formel I ist.

8. Verfahren gemäß Anspruch 1, wobei das hydroxylhaltige Lösungsmittel ein Niederalkanol umfasst.

9. Verfahren gemäß Anspruch 1, wobei das hydroxylhaltige Lösungsmittel Methanol, Ethanol, n-Propanol, Isopropanol, n-Butanol oder t-Butanol ist.

10. Verfahren gemäß Anspruch 1, wobei die Konzentration an Chlorwasserstoffgas in dem hydroxylhaltigen Lösungsmittel 1-20 % w/v ist.

11. Verfahren gemäß Anspruch 10, wobei die Konzentration an Chlorwasserstoffgas in dem hydroxylhaltigen Lösungsmittel 5-15 % w/v ist.

12. Verfahren gemäß Anspruch 1, wobei die Reaktionstemperatur im Bereich von -20°C bis 80°C ist.

13. Verfahren gemäß Anspruch 12, wobei die Reaktionstemperatur im Bereich von 0°C bis 30°C ist.

14. Verfahren gemäß Anspruch 1, wobei besagtes hydroxylhaltiges Lösungsmittel Chlorwasserstoffgas zu dem Zeitpunkt enthält, bei dem besagte Reaktanten darin gelöst werden.

15. Verfahren gemäß Anspruch 1, wobei besagtes Chlorwasserstoffgas zu der Reaktionsmischung gegeben wird, nachdem besagte Reaktanten in besagtem hydroxylhaltigem Lösungsmittel gelöst worden sind.

16. Verfahren gemäß Anspruch 1, wobei die 1,8-disubstituierten 1,3,4,9-Tetrahydropyran(3,4-b)-indol-1-Essigsäureester der Formel I durch Filtration wiedergewonnen werden.

17. Verfahren gemäß Anspruch 1, wobei die 1,8-disubstituierten 1,3,4,9-Tetrahydropyran(3,4-b)-indol-1-Essigsäureester der Formel I durch Extraktion der Reaktionsmischung mit einem Lösungsmittel, wobei besagtes Lösungsmittel die Eigenschaft hat, dass das gewünschte Produkt gemäß Formel I darin löslich ist und es keine homogene Mischung mit besagtem hydroxylhaltigen Lösungsmittel bildet, und anschließende Wiedergewinnung des Produktes gemäß Formel I durch Entfernung des Lösungsmittels wiedergewonnen werden.

18. Verfahren gemäß Anspruch 17, wobei besagtes Lösungsmittel aus Pentan und Hexan ausgewählt wird.

19. Verwendung des Verfahrens gemäß Anspruch 1 zur Herstellung von 1,8-disubstituierten 1,3,4,9-Tetrahydropyran(3,4-b)-indol-1-Essigsäureester der Formel I und deren anschließende Hydrolyse, um die entsprechenden Säuren wie Etodolac zu ergeben.

## Revendications

1. Procédé pour la préparation d'esters acétiques 1,8-disubstitués 1,3,4,9-tétrahydropyrano (3,4-b)-indole-1 de formate I : 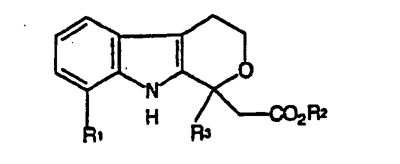 dans laquelle R₁ est choisi à partir du groupe composé d'hydrogène, d'alkyle inférieur et d'alcényle inférieur, R₂ est choisi à partir du groupe composé d'alkyle inférieur et d'aralkyle, R₃ est choisi à partir du groupe composé d'alkyle inférieur, d'alcényle inférieur, de cyclohexyle inférieur, de phényle et de benzyle, ledit procédé consistant à faire réagir 7-àlkyltryptophol de formule II : 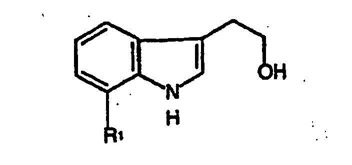 avec un β-kétoester de formule III : 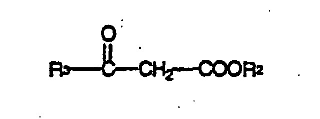 dans un solvant hydroxylique, comprenant au moins un système alcanol ayant 1-4 atomes de carbone, contenant du gaz de chlorure d'hydrogène, et subséquemment à récupérer le produit de formule I.

2. Procédé de la revendication 1, dans lequel R₂ est un alkyle inférieur dans le composé de formule I.

3. Procédé de la revendication 2, dans lequel R₂ est du méthyle dans le composé de formule I.

4. Procédé de la revendication 3, dans lequel R₁ est un alkyle inférieur dans le composé de formule I.

5. Procédé de la revendication 4, dans lequel R₁ est de l'éthyle dans le composé de formule I.

6. Procédé de la revendication 5, dans lequel R₃ est un alkyle inférieur dans le composé de formule I.

7. Procédé de la revendication 6, dans lequel R₃ est de l'éthyle dans le composé de formule I.

8. Procédé de la revendication 1, dans lequel le solvant hydroxylique comprend un alcanol inférieur.

9. Procédé de la revendication 1, dans lequel le solvant hydroxylique est du méthanol, de l'éthanol, du n-propanol, de l'isopropanol, du n-butanol ou du t-butanol.

10. Procédé selon la revendication 1, dans lequel la concentration du gaz de chlorure d'hydrogène dans un solvant hydroxylique est de 1-20% p/v.

11. Procédé selon la revendication 10, dans lequel la concentration du gaz de chlorure d'hydrogène dans un solvant hydroxylique est de 5-15% p/v.

12. Procédé selon la revendication 1, dans lequel la température réactionnelle est dans la plage de -20°C à 80°C.

13. Procédé selon la revendication 12, dans lequel la température réactionnelle est dans la plage de 0°C à 30°C.

14. Procédé de la revendication 1, dans lequel ledit solvant hydroxylique contient du gaz de chlorure d'hydrogène au moment où lesdits réactifs sont dissous dans celui-ci.

15. Procédé de la revendication 1, dans lequel ledit gaz de chlorure d'hydrogène est ajouté au mélange réactionnel après dissolution desdits réactifs dans ledit solvant hydroxylique.

16. Procédé de la revendication 1, dans lequel les esters acétiques 1,8-disubstitués 1,3,4,9-tétrahydropyrano(3,4-b)-indole-1 de formule I sont récupérés par filtration.

17. Procédé de la revendication 1, dans lequel les esters acétiques 1,8-disubstitués 1,3,4,9-tétrahjrdropyrano(3,4-b)-indole-1 de formule I sont récupérés en extrayant le mélange réactionnel avec un solvant, où ledit solvant a la caractéristique que le produit souhaité de formule I est soluble dans celle-ci et il ne forme pas un mélange homogène avec ledit solvant hydroxylique, et en récupérant par la suite le produit de formule I en éliminant le solvant.

18. Procédé de la revendication 17, dans lequel ledit solvant est choisi à partir de pentane et d'hexane.

19. Utilisation du procédé de la revendication 1 pour la préparation des esters acétiques 1,8-disubstitués 1,3,4,9-tétrahydropyrano(3,4-b)-indole-1 de formule I et leur hydrolyse subséquente pour fournir les acides correspondants tels que l'étodolac.
